# EUROPEAN PATENT SPECIFICATION

(11) **EP 1 085 912 B1**
(45) Date of publication and mention of the grant of the patent: **12.11.2003**
(21) Application number: 99927919.3
(22) Date of filing: 07.06.1999
(51) Int. Cl.: A61L 15/28

(54) **USE OF A WOUND DRESSING IN THE TREATMENT OF ACUTE WOUNDS**
VERWENDUNG VON WUNDVERBÄNDEN ZUR BEHANDLUNG VON AKUTEN WUNDEN
UTILISATION D'UN PANSEMENT POUR LE TRAITEMENT DE PLAIES VIVES

(30) Priority: 09.06.1998 GB 9812278
(43) Date of publication of application: 28.03.2001
(73) Proprietor: Bristol-Myers Squibb Company, New York, N.Y. 10154 (US)
(72) Inventor: KREIS, Robert Walter, NL-1942 LE Beverwijk (NL); VLOEMANS, Jos, NL-1942 LE Beverwijk (NL); DU PONT, Johannes, Sebastianus, NL-1942 LE Beverwijk (NL); HOEKSTRA, Matthias, Johannas, NL-1942 LE Beverwijk (NL)
(74) Representative: Mays, Julie
(86) International application number: EP9903951
(87) International publication number: WO99064079

(56) References cited:
- WO-A-94/16746
- US-A- 3 491 760
- US-A- 3 949 742
- US-A- 4 767 619

## Description

This invention relates to the use of a wound dressing for the treatment of burns and other acute wounds and in particular the use of a dressing as a replacement for a biological dressing in the treatment of an acute wound.

Acute wounds which result in loss of skin, such as burns, require treatment in a variety of ways depending on size, severity and location. For instance a burn in which only the outer layer of skin is burned over a small percentage of the total skin surface can be treated with first aid measures in the home. There are many kinds of dressing available for these types of burns.

With burns in which perhaps all the layers of skin are damaged, and sometimes fat, nerve, muscle and tendon are involved or where a large percentage of the total skin surface is damaged it is usually necessary to use some form of skin graft or biological dressing to cover the wound and aid healing.

It is not always possible to take skin grafts from the patient (autograft) due to the extent of burning. In such circumstances biological dressings are the only alternative. Such dressings have many functions and take many forms. Some of their functions include preventing desiccation of the wound surface, decreasing evaporative water loss, decreasing heat loss, reducing bacterial proliferation, decreasing wound pain, protecting exposed tendons and nerves, and enhancing healing. Examples of biological dressings include naturally occurring tissues such as cutaneous allografts, cutaneous xenografts or amniotic membranes; skin substitutes such as synthetic laminates, collagen based composites or collagen based dermal analogs; and culture derived tissue such as cultured autologous keratinocytes and fibroblast seeded dermal analogs.

Biological dressings can also be used as temporary covers over wounds that are subsequently covered by culture derived grafts and over wounds that have been treated with cutaneous widely expanded mesh grafts or culture derived grafts which leave open wound areas to achieve wound closure.

Biological dressings are sophisticated and therefore tend to be expensive and can carry the same risks of cross-contamination that are encountered with blood and blood products.

There therefore exists a need for a dressing that can be used to treat acute wounds, which performs in a similar manner to a biological dressing but mitigates the disadvantages of high cost and risk of contamination encountered with biological dressings.

Surprisingly we have found that certain wound dressings known for use in other treatments, such as the treatment of chronic wounds, can behave like a biological dressing and can reduce the need for autograft.

Accordingly the invention provides the use of a wound dressing for the preparation of a substitute for a biological dressing for use in the treatment of acute wounds and in particular the treatment of wounds which contain epithelial remnants like partial thickness burns.

Such wound dressings do not have the disadvantages of high cost and cross contamination that may be encountered with biological dressings.

We have found that a wound dressing, to be suitable as a substitute for a biological dressing preferably is adherent to the wound without preventing the outgrowth of the epithelium. This is truly surprising since conventional wisdom teaches that wound dressings should not adhere to the acute wound and many known dressings are provided with measures to avoid adherence such as being impregnated with paraffin or being coated with silicone. We have found that an adherent dressing has advantages over the prior art dressings which allow the dressing to be used in those situations where a biological dressing would otherwise be used.

We have also found that wound dressings suitable as replacements for biological dressings preferably promote the migration of enzymes, neutrophils, fibroblasts and cellular debris into the dressing. Whilst not wishing to be bound by theory we believe that this migration, which we term as "vertical wicking", modulates the inflammatory response of the wound and contributes to successful healing of the wound.

Accordingly the invention provides the use of a wound dressing for the preparation of a substitute for a biological dressing for use in the treatment of acute wounds by adhering to the wound and providing conditions conducive to epithelial outgrowth.

According to a further aspect, the invention provides the use of a wound dressing for the preparation of a substitute for a biological dressing for use in the treatment of acute wounds and particularly partial thickness burns by promoting vertical wicking into the dressing and thereby modulating inflammatory response.

In particular we have found that certain fibrous wound dressings are suitable for use in the present invention. The wound contact layer is fibrous and can comprise fibres of alginate, viscose, modified cellulose, cellulose, polyester, polypropylene and co-polymers thereof, pectin, chitosan fibres, hyaluronic acid fibres or other polysaccharide fibres or fibres derived from gums. Most preferred are highly absorbent fibres such as, modified cellulose fibres as described in WO93/12275 to Courtaulds Plc or WO 94/16746 to Courtaulds Plc and alginate fibres as described in WO 94/17227 to E.R. Squibb and Sons. By "highly absorbent" with respect to the fibre it is meant that they can absorb at least 25 g/g of deionized water. The fibres for use in the wound layer may also be mixed or blended to form a composite layer or may be fibres made of a mixture of any of the above ingredients. Preferably fibrous wound dressings include those described in WO94/16746 to Courtaulds Plc which discloses wound dressings made from carboxymethyl cellulose filaments.

It is particularly surprising that a fibrous dressing has this effect because biological dressings are occlusive in order to provide a barrier to bacteria. A fibrous dressing has an open structure and therefore would not be expected to behave like a biological dressing.

A wound dressing made from carboxymethyl cellulose filaments is marketed as Aquacel® ex ConvaTec for use in the treatment of chronic wounds such as ulcers or pressure sores. We have observed that when Aquacel® is used on burns it exhibits surprising behaviour in that it adheres to the wound bed without blocking epithelial outgrowth. This type of behaviour would usually only be seen with a biological dressing such as allograft.

We have observed that Aquacel® effectively absorb the polymorfonuclear infiltrate (pmn's), which invades the wound bed just after wounding. The pmn-infiltrate is an important part of the defense mechanism of the body against infection. Based on the optimal swelling capacity of the fibres of the wound dressing, the pmn-infiltrate becomes mainly separated from the wound bed, which is invaded after a couple of days by macrofages as major orchestrator of repair mechanism of the body. Because the wound dressing is adherent to the wound bed, there is an optimal protection against bacterial infection; also the pmn-infiltrate still shows enzymatic activity once located in the dressing material. Epithelialization continues undisturbed as a result of further population of the wound bed by macrophages. In addition by absorption and isolation of the pmn-infiltrate by this specific dressing material the remaining dermal matrix is protected against further damage by enzymes of the pmn's.

In the context of the present invention, biological dressings are: naturally occurring tissues such as cutaneous allografts, cutaneous xenografts or amniotic membranes; skin substitutes such as synthetic laminates, collagen based composites or collagen based dermal analogs; and culture derived tissue such as cultured autologous keratinocytes and fibroblast seeded dermal analogs.

Preferably the wound dressing of the present invention is used on acute wounds which are forming exudate. These tend to be partial thickness burns.

The invention will now be illustrated by way of the following non-limiting examples.

### Example 1

### Comparison of Aquacel with a biological dressing on second degree burns

Dressing materials used for the treatment of second degree burns ideally fulfill a number of demands. In addition to pain reduction, prevention of dehydration and infection, minimising the risk of hypertrofic scarring is an important feature. Cadaver allograft skin provided by the Euro Skin Bank was applied to the wound bed of second degree burns on the day of the burn or the first day post burn. In approximately 60% of burns treated the allograft became adherent to the wound, thereby reducing the risk of infection, regulated fluid loss and dried out to form a crust as wound healing was in progress.

After 14 days the allograft was removed to reveal pale pink skin which had no signs of an inflammation reaction.

Aquacel® was used on 58 patients with second degree burns. It was applied to the wound on the first day post burn and in 80% to 90% of burns treated became adherent to the whole wound surface. Aquacel® dried out to form a crust as wound healing progressed and was easily peeled off once the wound had healed. Patients reported no pain or disruption of the newly formed skin. The healed skin had a stable, pale pink appearance with no signs of inflammation.

These results show the similarity in action of Aquacel® to allograft skin. They also show that the incidence of Aquacel® becoming fully adherent to the wound was greater than that with allograft skin which makes Aquacel® a more reliable treatment for wounds than allograft skin.

### Example 2

Partial thickness wounds were made on the back of male Wistar rats. The wounds were covered with Aquacel and fixed in place with silk tape. After 3 to 7 days the rats were sacrificed and the wound tissues frozen in liquid nitrogen so that cryosections could be prepared. The cryosections were stained with haematoxilin-eosine so that the wound healing process could be evaluated. Specific immuno-histological sections were prepared to identify macrophages.

During the healing process, Aquacel® adhered very well to the wounds. After four days, Aquacel had the form of a moist gel. Once re-epithelialization was complete, the Aquacel® became dry and could be easily removed.

The cryosections showed that the fibres of Aquacel® had fully swelled leaving no interfiber spaces. This suggests that Aquacel® had vertically wicked the wound exudate away from the wound along with cellular debris and enzymes. We believe that this property of vertical wicking creates an environment where the inflammatory response of the wound is modulated and this provides optimal conditions for the outgrowth of the epithelium and wound healing.

This theory is supported by the fact that there were no signs of infection in the wound and bacteria were not observed in the cryosections.

### Example 3

### Use of Aquacel as a temporary cover over excised and skin transplanted wounds

Patients with extensive burns are often treated with autologous expanded mesh skin transplants. As these are susceptible to desiccation and infection the wound area is "closed" by the use of split skin allografts over the autologous transplants. Allografts are often not available and have the disadvantages of cost and contamination risk. As an alternative to allografts, synthetic biological dressings have been used but these often disrupt the outgrowth of the epithelium, a phenomenon known as blocking.

In several patients it was observed that the excision of burned tissue and transplantation with either autologous skin micrografts or skin meshgrafts, could be combined with Aquacel® as a temporary covering material. With Aquacel® no blocking of the outgrowth of the epithelium was observed.

These results show the superior performance of Aquacel® when used as a replacement for a biological dressing.

## Claims

1. Use of highly absorbent fibres that can absorb at least 25g/g of deionized water and are alginate, viscose, modified cellulose, cellulose, polyester, polypropylene and co-polymers thereof, pectin, chitosan fibres, hyaluronic acid fibres or other polysaccharide fibres or fibres derived from gums, in the manufacture of a wound dressing which becomes adherent to the wound for use in the treatment of burns.

2. Use of highly absorbent fibres that can absorb at least 25g/g of deionized water and are alginate, viscose, modified cellulose, cellulose, polyester, polypropylene and co-polymers thereof, pectin, chitosan fibres, hyaluronic acid fibres or other polysaccharide fibres or fibres derived from gums, in the manufacture of a wound dressing for use in the treatment of burns by adhering to the wound and providing conditions conducive to epithelial outgrowth.

3. Use of highly absorbent fibres as claimed in any preceding claim **characterised in that** the wound contact layer of the dressing is fibrous.

4. Use of highly absorbent fibres as claimed in any preceding claim **characterised in that** the dressing is used on partial thickness burns.

5. Use of highly absorbent fibres as claimed in any preceding claim **characterised in that** the fibres are modified cellulose fibres.

## Patentansprüche

1. Verwendung hochabsorbierender Fasern, welche mindestens 25g/g entionisiertes Wasser absorbieren können und aus Alginat, Viskose, modifizierter Cellulose, Cellulose, Polyester, Polypropylen und Copolymeren davon, Pectin, Chitosanfasern, Hyaluronsäurefasern oder anderen Polysaccharidfasern oder von Gummiharzen abgeleitete Fasern sind, zur Herstellung eines auf der Wunde haftenden Wundverbandes zur Verwendung bei der Behandlung von Verbrennungen.

2. Verwendung hochabsorbierender Fasern, welche mindestens 25g/g entionisiertes Wasser absorbieren können und aus Alginat, Viskose, modifizierter Cellulose, Cellulose, Polyester, Polypropylen und Copolymeren davon, Pectin, Chitosanfasern, Hyaluronsäurefasern oder anderen Polysaccharidfasern oder von Gummiharzen abgeleitete Fasern sind, zur Herstellung eines Wundverbandes zur Verwendung bei der Behandlung von Verbrennungen durch Wundhaftung und Schaffung von epithelwuchsfördernden Bedingungen.

3. Verwendung hochabsorbierender Fasern gemäß einem der vorstehenden Ansprüche, **dadurch gekennzeichnet, dass** die mit der Wunde in Kontakt stehende Schicht des Verbandes faserförmig ist.

4. Verwendung hochabsorbierender Fasern gemäß einem der vorstehenden Ansprüche, **dadurch gekennzeichnet, dass** der Verband bei Verbrennungen 2. Grades verwendet wird.

5. Verwendung hochabsorbierender Fasern gemäß einem der vorstehenden Ansprüche, **dadurch gekennzeichnet, dass** die Fasern modifizierte Cellulosefasern sind.

## Revendications

1. Utilisation de fibres hautement absorbantes pouvant absorber au moins 25 g/g d'eau déminéralisée et qui sont de l'alginate, du viscose, de la cellulose modifiée, de la cellulose, du polyester, du polypropylène et ses copolymères, de la pectine, des fibres de chitosane, des fibres d'acide hyaluronique ou d'autres fibres de polysaccharide ou des fibres dérivés de gommes, dans la fabrication d'un pansement qui devient adhérent à la plaie pour utilisation dans le traitement des brûlures.

2. Utilisation de fibres hautement absorbantes pouvant absorber au moins 25 g/g d'eau déminéralisée et qui sont de l'alginate, du viscose, de la cellulose modifiée, de la cellulose, du polyester, du polypropylène et ses copolymères, de la pectine, des fibres de chitosane, des fibres d'acide hyaluronique ou d'autres fibres de polysaccharide ou des fibres dérivés de gommes, dans la fabrication d'un pansement destiné à l'utilisation dans le traitement des brûlures en adhérant à la plaie et en fournissant des conditions conduisant à la croissance de l'épithélium.

3. Utilisation de fibres hautement absorbantes comme revendiquées selon une quelconque des revendications précédentes **caractérisée en ce que** la couche de contact avec la plaie du pansement est fibreuse.

4. Utilisation de fibres hautement absorbantes comme revendiquées selon une quelconque des revendications précédentes **caractérisée en ce que** l'on utilise le pansement sur des brûlures d'épaisseur partielle.

5. Utilisation de fibres hautement absorbantes comme revendiquées selon une quelconque des revendications précédentes **caractérisée en ce que** les fibres sont des fibres de cellulose modifiée.
